(19) Europäisches Patentamt
European Patent Office
Office européen des brevets

(11) EP 2 617 698 A1

(12) # EUROPEAN PATENT APPLICATION

(43) Date of publication:
**24.07.2013 Bulletin 2013/30**

(51) Int Cl.:
***C07C 17/08*** (2006.01) ***C07C 21/06*** (2006.01)

(21) Application number: **12173956.9**

(22) Date of filing: **27.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SOLVAY SA**
**1120 Bruxelles (BE)**

(72) Inventors:
- **Strebelle, Michel**
**1150 Brussels (BE)**
- **Liebens, Armin T.**
**1200 Brussels (BE)**
- **Balthasart, Dominique**
**1120 Brussels (BE)**
- **Piccinini, Marco**
**1020 Brussels (BE)**

(74) Representative: **Vande Gucht, Anne et al**
**Solvay S.A.**
**Intellectual Assets Management**
**Rue de Ransbeek, 310**
**1120 Bruxelles (BE)**

(54) **Process for the hydrohalogenation of an unsaturated hydrocarbon and for the manufacture of vinyl chloride by hydrochlorination of acetylene**

(57) Process for the hydrohalogenation of an unsaturated hydrocarbon using a catalyst comprising at least one ionic liquid (IL) and a metal, according to which said IL and metal are supported on a solid carrier, said process being a continuous industrial process wherein the overall unsaturated hydrocarbon conversion is of at least 60 % during at least 500 hours.

EP 2 617 698 A1

Printed by Jouve, 75001 PARIS (FR)

## Description

The present invention relates to a process for the hydrohalogenation of an unsaturated hydrocarbon and for manufacturing vinyl chloride by hydrochlorination of acetylene. The manufacture of vinyl chloride by reaction between acetylene and hydrogen chloride is conventionally carried out in the gas phase, in a fixed-bed reactor, in the presence of a heterogeneous solid catalyst based on mercury chloride on a support. Mainly for reasons of toxicity, there is currently an increasing interest in catalytic systems with decreased mercury content or which are free of mercury compounds.

Various catalysts intended to replace the current catalysts in gas-phase processes have been developed.

For example, unexamined Japanese Patent Application 52/136104 describes a process of hydrochlorinating acetylene in the gas phase in the presence of a fixed catalyst bed composed of noble metal halides deposited on active carbon. To date however, the lifetime of such alternative catalysts intended for gas-phase processes remains much shorter than that of catalysts based on mercury compounds.

Furthermore, in the literature there are some examples of hydrochlorinating acetylene in the presence of a liquid catalytic medium.

German Patent 709.000 describes a process for preparing vinyl halides by bringing acetylene into contact, at high temperatures, with a molten mass of hydrohalide salts of organic bases containing a standard catalyst. Aliphatic, aromatic or heterocyclic amines and mixtures thereof are envisaged as organic bases.

Inventor's certificate SU 237116 describes the use of an aqueous acid solution containing 46 wt % of cuprous chloride and from 14 to 16 wt % of a methylamine, dimethylamine or trimethylamine hydrochloride.

European Patent Application EP-A-0 340 416 discloses a process for preparing vinyl chloride by reaction of acetylene with hydrogen chloride in the presence of a palladium compound as catalyst in a solvent composed of an aliphatic or cycloaliphatic amide, at a temperature above room temperature. Although it allows high yields to be obtained, this process has, however, some significant drawbacks : it has emerged that, under the reaction conditions, the liquid catalyst system gradually degrades, forming blackish products of carbonaceous appearance. In addition, in the presence of hydrogen chloride, the amide is converted to a hydrochloride, the melting point of which is generally much higher than room temperature. N-Methylpyrrolidone hydrochloride, for example, is only liquid above 80°C. In practice, this may cause serious implementation problems, problems linked to agglomeration of the catalytic medium during reactor shutdowns or blocking of the lines at the coldest points of the installation. The entire reactor and also the lines in which the reaction medium flows must then be continuously kept at a temperature above the melting point of the hydrochloride.

These various problems seemed to have been solved thanks to the catalytic hydrochlorination systems described in European Patent Applications EP 0 519 548-A1 and EP 0 525 843-A1 and which comprise at least one group VIII metal compound and either an amine hydrochloride, the melting point of which is less than or equal to 25°C, or a fatty amine hydrochloride comprising more that 8 carbon atoms, the melting point of which is above 25°C and an organic solvent chosen from aliphatic, cycloaliphatic and aromatic hydrocarbons and mixtures thereof. Nevertheless, the catalyst systems that are described therein, especially those of which the group VIII metal compound is platinum (II) chloride or palladium (II) chloride, are not completely satisfactory when considering the performances that they enable to be achieved in terms of productivity of the vinyl chloride produced by hydrochlorination of acetylene and in terms of long term stability.

WO 2008/77868 discloses a catalytic hydrochlorination system comprising at least one amine hydrochloride and at least one group VIII metal compound selected from the group composed of mixtures of a platinum (IV) compound with Sn(II) chloride, mixtures of a platinum (II) compound with triphenylphosphine oxide and mixtures of a palladium (II) compound with triphenylphosphine. These catalytic systems show an improved productivity compared to the systems as described in European patent applications EP-A 0519548 and EP-A 0525843.

Finally, patent application CN 101716528 (citing Zhiyong Yu as inventor) discloses catalytic systems for production of vinyl chloride by the hydrochlorination of acetylene comprising an imidazolium (which is a non-protonated cation)-based ionic liquid (IL) with chloride, bromide, hexafluorophosphate or tetrafluorophosphate ion as anion and one or more of gold, platinum, palladium, tin, mercury, copper or rhodium chlorides.

The last above-mentioned catalytic system seems to lead to relatively high selectivity and conversion rates at least when it is used in lab apparatus made of chemically inert material (like glass or Pyrex). In that regard, publication Green Chem., 2011, 13, 1495 having Zhiyong Yu as one of the authors, explicitly describes a glass reactor without mentioning any corrosion problem. In fact, glass was not chosen on purpose for corrosion problems but was merely the fall back material used for general purpose lab apparatus.

However, the Applicant noticed that surprisingly, the materials used in the industrial facilities handling the processes of the above mentioned patents using an amine hydrochloride, were rapidly and severely corroded if submitted to the above described imidazolium containing hydrochlorination medium. And from some testing, the Applicant noticed that this problem seems to be generalized at least to ILs comprising at least one non-protonated cation and HCl.

Hence, a co-pending application to the Applicant (PCT/EP2012/052911) aims at protecting a process for the hydrohalogenation of an unsaturated hydrocarbon (preferably acetylene) using an IL as catalyst and which is at least partly carried out in apparatus made from or covered with materials which are resistant to halogenated acids in dissociated

form.

On the other hand, several articles have been published in the recent years (namely on the internet) regarding the use of Supported Ionic Liquid Phase (SILP) catalysts in low temperature water-gas shift reactions and in hydroformylation and carbonylation reactions.

The Applicant has now found that surprisingly, such SILP catalysts do not only reduce significantly the above mentioned corrosion problem during the hydrohalogenation of an unsaturated hydrocarbon using an IL catalyst but additionally, they allow a tremendous increase in the unsaturated hydrocarbon conversion rate (which can even reach almost 100 % in some circumstances) and in the productivity of the catalyst.

Therefore, the present invention relates to a process for the hydrohalogenation of an unsaturated hydrocarbon using a catalyst comprising at least one ionic liquid (IL) and a metal, according to which said IL and metal are supported on a solid carrier, said process being a continuous industrial process wherein the overall unsaturated hydrocarbon conversion is of at least 60 % during at least 500 hours.

Preferably, the overall unsaturated hydrocarbon conversion over at least 500 hours is of at least 80 % and even, of at least 90 %, and even above 95 % or 99 % in some circumstances. By "overall" is meant that if more than one reactor is used, the conversion of the unsaturated hydrocarbon from the entry of the first reactor to the exit of the last reactor, if they are in series, or from the entry of the reactors to their exit, if they are in parallel, should be of at least 60 %, preferably of at least 80 % and even more preferable : of at least 90 % (and even above 95 % or 99 % in some circumstances) and this over a duration of at least 500 h, preferably at least 1000 h and even more preferably, at least several days, preferably months or even years. The reaction could be realized in a single reactor, in more than one reactor, or in any combination of any number of reactors with one or more unit operation such as quenching, distillation, evaporation. When the reaction is realized in any number of reactors in combination with one or more unit operation, a distillation unit is preferred. Particularly preferred are reactive distillation units. In case of combination, the overall unsaturated hydrocarbon conversion is meant the conversion between the feed of the combined unit operations and the exit thereof.

As to the overall selectivity of the catalyst according to the invention, it is preferably of at least 90 %, more preferably of at least 95 % and even more preferably : of at least 99 %, for at least the same periods of time as mentioned above (500 h, 1000 h, days, months, years). The selectivity is meant here as the ratio of the halogenated product (like VCM) produced to the unsaturated hydrocarbon (like acetylene for VCM) reacted, which means for the example of acetylene to VCM : selectivity = [(VCM)out-(VCM)in]/[(C2H2)in-(C2H2)out] where (X) is the molar flow of the component X.

A parameter which may be important from an industrial point of view is the yield or the product of the unsaturated hydrocarbon conversion and of the selectivity. The yield is usually over 54 %, preferably over 72 % more preferably over 81 % particularly over 90 %.

Finally, the overall productivity of said catalyst preferably also is above a given value which depends on the unsaturated hydrocarbon content of the unsaturated hydrocarbon source.

When the unsaturated hydrocarbon source has a high unsaturated hydrocarbon content (generally equal to or above 10 %, preferably 15 % and even more preferably, 20 % mol of the flux), the productivity preferably is of at least 100, more preferably at least 500, more preferably at least 1000 expressed in g of hydrohalogenated component produced (like VCM) per liter of catalyst and per hour (or gVCM/l catalyst/h) again for the same periods of time.

If the unsaturated hydrocarbon content of the unsaturated hydrocarbon source is low (typically, below 10 %, even below 5 % or even below 1 % mol of the flux), the productivity preferably is of at least 1 (again expressed in g of hydrohalogenated unsaturated hydrocarbon per liter of catalyst and per hour), more preferably of at least 5 and even more preferably of at least 10.

The volume of catalyst is to be understood as being the total volume of the support bearing the IL and the metal. It is calculated as follows :

First the apparent density ("d") of the catalyst is measured. The mass ("m") of a precise volume of catalyst ("V") of about 3 to 10 mL is determined using a lab balance. The density is then calculated as :

$$d = \frac{m}{V}$$

This allows the conversion of the initial mass of catalyst used for the reaction into a volume unit.

Second, the amount (in g) of halogenated component produced per hour, is calculated as follows, taking the example of the halogenation of acetylene in VCM:

In a first step, the $m^3$VCM/h is calculated according to the following equation :

$$m^3VCM / h = f_{C2H2}^{IN} \cdot C_{C2H2} \cdot S_{C2H2}$$

where "f" represent the inlet flow rate of acetylene expressed in $m^3/h$, "C" the conversion of acetylene and S the selectivity in VCM. The value obtained is then transformed into molVCM/h using the ideal gas law :

$$pV = nRT$$

where "p" is pressure (101325 Pa), "V" the flow rate expressed as $m^3/h$, "n" the molar flow rate expressed as mol/h, "R" the ideal gas constant (~8.314 $m^3$ Pa $K^{-1}$ $mol^{-1}$) and "T" the temperature expressed in Kelvin. The molVCM/h is then multiplied by the molecular weight of VCM to obtain gVCM/h.

This value is then divided by the catalyst volume expressed in liter in order to obtain the productivity as expressed above.

Although all the embodiments described below allow to reach the conversion rate claimed in the present invention and eventually the selectivity and/or productivity ranges set forth above, they are believed to bring other advantages (each of them alone or combined with at least one of the others) so that they could each be claimed as invention(s) per se. They namely allow getting very low corrosion rates on materials which are normally completely dissolved in IL (like Zr for instance) so that said materials can be used for industrial apparatus during a normal industrial life time (several years, typically at least 10 years) at the design pressure either in massive form or as a protective layer allowing the apparatus to resist to the corrosivity of the reaction medium. The protective layer could be made of the same material as the one providing the mechanical resistance of the apparatus or from another material. Exemples of materials that can be used either in massive form or as protective layer are metals, graphite, fluorinated polymers and silicon carbide. Examples of materials that may be used as protective layer (made of a different material than the one providing the mechanical resistance) are metals such as Nb, Ta, metal alloys such as Hastelloy ® C276, Hastelloy ® HB2, Monel ®, Inconnel ®, Incoloy ®, enamels such as Pfaudler®email WWG, Pfaudler®email 4300, Pfaudler®email ASG, fluorinated polymers like PTFE, PFA, MFA, PVDF...

For metals, the corrosion allowance (or amount of corrosion allowed before having to replace the concerned piece) is usually less than 5 mm, a corrosion allowance of less than 3 mm being preferable, a corrosion allowance of less than 2 mm being even more preferable and a corrosion allowance of less than 1,8 mm being particularly preferred. Corrosion allowance for metals is usually higher than 0,01 mm, preferably than 0,03 mm and even more preferably than 0,05 mm. For non-metallic protective layers, the thickness of the layer is usually greater than 0,1 mm, preferably than 0,3 mm, even more preferably 0,5 mm. Usually the thickness of the protective layer is lower than 20mm, preferably lower than 15 mm and even more preferably than 10mm. The apparatus could be made in a single material or be bimaterial. When the apparatus is bimaterial, generally the support material is not in contact with the reaction medium and provides the mechanical resistance. The support material could be metallic or plastic. Example of support materials are carbon steel, stainless steel, fiber glass reinforced polyester, polyethylene, polypropylene, PVC... Carbon steel is preferred. The support material providing the mechanical resistance is usually subjected to the corrosivity of the external atmosphere. Preferably the support material also has a protective layer versus the external corrosion. External protective layers could be an extra thickness of the same material, or a paint layer. When the protective layer is an extra thickness it is usually higher than 0,1 mm, preferably than 0,3, and even more preferably than 0,5 mm, while it usually is lower than 5mm, preferably than 3mm, even more preferably than 2,5 mm.

Any unsaturated hydrocarbon may be used in the process according to the invention. Preferably, it is gaseous at the hydrohalogenation temperature. By unsaturated hydrocarbon is meant a component made of carbon and hydrogen and having at least one double and/or triple bond between to C atoms ; examples are acetylene, ethylene, and the like. Acetylene gives good results within the frame of the invention.

In the present description, the term "acetylene" has to be understood as a source of acetylene which may either be "pure" acetylene (as available commercially) or mixtures comprising acetylene which can, in addition to acetylene, comprise other components which may be by-products of acetylene synthesis, e.g. ethylene or other unsaturated hydrocarbons, gases like N2, CO2, H2, CO, H2O ... The origin of such mixtures of different unsaturated compounds can be any known source of reaction mixtures as they may be obtained in the course of the known synthesis methods for acetylene. Mixtures comprising less than 50 % of acetylene can be used. Preferably however, the term "acetylene" refers to mixtures comprising at least 90 % of acetylene and more preferably 99 % of acetylene, or even 99.9 % acetylene.

Acetylene is mainly manufactured by the partial combustion of methane, by oxidative cracking of hydrocarbon source, by electric arc coal furnace, by plasma coal furnace or appears as a side product in the ethylene stream from cracking of hydrocarbons.

Another method for the manufacture of acetylene is the hydrolysis of calcium carbide

$$CaC_2 + 2H_2O \rightarrow Ca(OH)_2 + C_2H_2$$

The manufacture of CaC2 from CaO or CaCO3 and C requires extremely high temperatures of approximately 2000°C, necessitating the use of an electric furnace or the like.

In a preferred embodiment of the invention, the hydrohalogenation reaction is hydrochlorination. This embodiment is particularly interesting when the unsaturated hydrocarbon is acetylene.

Mixtures comprising acetylene and ethylene may be used directly as such, i.e. without the necessity to separate the components as the reactivity of acetylene vs. ethylene should enable the hydrochlorination of acetylene to be carried out first with separation of the vinyl chloride obtained and the subsequent use of ethylene. This ethylene could be chlorinated to produce 1,2-dichoroethane (DCE) for a combined process for the manufacture of vinyl chloride monomer (VCM). The pyrolysis of the 1,2-dichloroethane can produce the hydrogen chloride for the first reaction with acetylene.

In one embodiment, the process of the invention is carried out after the pyrolysis of DCE in a process to produce DCE and VCM from ethylene, as an alternative to the hydrogenation of acetylene sometimes used in such a process.

In a first sub-embodiment, the catalyst of the invention is put within or downstream of a quenching device used to quench the pyrolysis gases.

In a second sub-embodiment of the invention, the catalyst of the invention is put in the column used to separate HCl from VCM and the other constituents of the quenched gases.

In a third sub-embodiment, the catalyst of the invention is put on a side-stream taken on the column used to separate HCl from VCM and the other constituents of the quenched gases. The side-stream is taken from any location of the column under or over the feed point, preferably over the feed point. The side-stream could be taken from the vapor phase or from the liquid phase or from both phases circulating in the column. Preferably the side-stream is taken from the vapor phase. The reacted gas (i.e. the gas from which the acetylene has been converted to VCM) is recycled downstream of the pyrolysis in any suitable place. Suitable recirculation place are for instance the quench, the feed of the column or the column itself.

In a fourth sub-embodiment, the catalyst of the invention is put on the stream separated at the top of the column, preferably after suitable thermal treatment and/or pressure treatment of said stream.

In these embodiments, the catalyst is preferably of the honeycomb type and preferably has large channels so as to avoid clogging of said channels by tars.

Depending on the acetylene content of the acetylene source, the hydrochlorination reaction can advantageously be carried out at a temperature in the range of from -30°C to 230°C. At higher temperatures, the catalytic system has a tendency to degrade.

The preferred reaction temperature, that is to say that offering the best compromise between productivity, yield and stability of the catalytic medium, is greater than or equal to about 40°C if the acetylene content of the acetylene source is high (see above for the meaning of these terms). The best results are then obtained at temperatures greater than or equal to about 50°C with a more particular preference for temperatures greater than or equal to about 80°C and a most particular preference for temperatures greater than or equal to about 110°C. Preferably, the reaction temperature does not exceed about 200°C. A reaction temperature of about 40°C to about 200°C is most particularly preferred. In certain cases a reaction temperature not exceeding 170°C has proven advantageous or even : not exceeding 130°C. Lower corrosion rates and coke formation are observed at lower temperatures, however, higher conversion rates may be observed at higher temperature.

When the acetylene content of the acetylene source is low (see above for the meaning of these terms), the hydrochlorination reaction can advantageously be carried out between -30°C and 200°C.

When the acetylene content of the acetylene source is high, the hydrochlorination reaction is advantageously carried out at atmospheric pressure or at higher pressures compatible with the safety regulations for handling acetylene. Usually the pressure will not exceed 10 Mpa, preferably not 5 MPa, and even more preferably it will not exceed 2.5 MPa.

When the acetylene content of the acetylene source is low, the acetylene partial pressure is usually less than 5 MPa, preferably than 2,5 and even more preferably than 1 MPa. The acetylene partial pressure is however preferably higher than 5 Pa, preferably than 8 Pa and even more preferably 10 Pa.

The hydrochlorination of acetylene could be carried out in the gaseous or in the liquid phase, preferably in the gaseous phase. The hydrochlorination of acetylene is advantageously carried out by bringing the gaseous reactants - acetylene and hydrogen chloride - into contact with the catalyst, in any suitable reactor.

The hydrochlorination reaction may be carried out conventionally in any equipment promoting gas or liquid contact on solid materials. Such equipments are entrained bed, pneumatic transportation, cyclone, fluidized bed, vibrating bed, fixed bed, moving bed, bubbling bed, spouted bed or any combination.

In a first embodiment, the reaction is carried out in a fixed bed and/or in pre-assembled structures. Equipment allowing to carry a fixed could be random fixed bed, structured fixed bed, catalytic structured packing, honeycomb

structure and the like or any combination of these equipments. In such equipment, the external surface to volume ratio (S/V) of the catalyst is usually chosen to be lower than or equal to 6 $10^4$ $m^{-1}$, preferably than 3 $10^4$, more preferably than 2 $10^4$ $m^{-1}$. In such equipment, the external surface to volume ratio is usually chosen to be higher than 10 $m^{-1}$, preferably than 20 and even more preferably 25 $m^{-1}$.

In this first embodiment, the process of the invention preferably comprises feeding continuously a reaction zone comprising the catalyst with at least the unsaturated hydrocarbon and the hydrohalogenation reactive, both in gaseous form, at a total linear velocity higher than or equal to 0.005 m/s, preferably 0.008 m/s, 0.01, even more preferably 0.02 m/s and lower than or equal to 20 m/s, preferably 15 m/s, even more preferably 12 m/s, and with a pressure drop across the reaction zone which is preferably lower than or equal to 50 kPa/m, more preferably than 40 kPa/m and even more preferably than 35 kPa/m.

In a second embodiment, the reaction is carried out in a fluidized flow. Equipment allowing to carry a fluidized flow could be fluidized bed, moving bed, vibrating bed, spouted bed, bubbling bed, and the like or any combination of these equipment. In such equipment, the external surface to volume ratio (S/V) of the catalyst is usually chosen to be lower than or equal to 1 $10^5$ $m^{-1}$, preferably than 8 $10^4$, more preferably than 5 $10^4$ $m^{-1}$. In such equipment, the external surface to volume ratio is usually chosen to be higher than 100 $m^{-1}$, preferably than 200 and even more preferably than 250 $m^{-1}$.

In this second embodiment, the total linear velocity is preferably higher than or equal to 0.15 m/s, preferably 0.25 m/s, even more preferably 0.4 m/s and lower than or equal to 6 m/s, preferably 4 m/s, even more preferably than 3 m/s, wherein the pressure drop across the reaction zone is preferably lower than or equal to 100 kPa/m, more preferably than 60 kPa/m.

In a third embodiment, the reaction is carried out in an entrained flow. Equipment allowing to carry an entrained flow could be pneumatic transportation, entrained bed, circulating bed, cyclone and the like or any combination of these equipments. In such equipment, the external surface to volume ratio (S/V) of the catalyst is usually chosen to be lower than or equal to 2 $10^6$ $m^{-1}$, preferably less than 1.2 $10^6$, more preferably than 6 $10^5$ $m^{-1}$. In such equipment, the external surface to volume ratio is usually chosen to be higher than 100 $m^{-1}$, preferably than 200 more preferably than 250 $m^{-1}$.

In this third embodiment, the total linear velocity is preferably higher than or equal to 0.25 m/s, preferably 0.4 m/s, even more preferably 0.5 m/s and lower than or equal to 20 m/s, preferably 15 m/s, even more preferably than 12 m/s, wherein the pressure drop across the reaction zone is preferably lower than or equal to 50 kPa/m, more preferably than 20 kPa/m and even more preferably than 5 kPa/m.

In the present invention, fixed bed and fluid bed are preferred, the fixed bed being most preferred.

In the embodiments above, the volume of the catalyst is understood to mean the geometric volume of the macroscopic catalyst at rest.

The "external surface" of the catalyst is understood to mean the surface of the catalyst particles constituting the macroscopic catalyst at rest. The external surface does not include the surface of the catalyst particles due to a possible macro-, meso- and/or microporosity of the catalyst particles.

Catalyst particles are intended to mean solid element of catalyst such as powders, extrudates, pellets, etc., honeycomb structures, catalytic micro-reactors and structured packings like Katapack®, Melapack®, etc. The catalyst can be a bulk catalyst or a supported catalyst.

The catalyst particles could be assembled in sub-structures such as honeycomb structures, catalytic micro-reactors, structured packing elements and the like ; individual particles could also be assembled in an ordered way such as in bed ; or individual particles could be manipulated as a whole in a fluidised bed, entrained bed, vibrating bed, moving bed, spouted bed, bubbling bed or the like.

When the catalyst particles are assembled in sub-structures, the external volume of the catalyst can be calculated from the average geometric outer dimensions of the catalyst sub-structures, using classical surface and volume formulas. The catalyst surface can be calculated from the average geometric dimensions of the outer and inner dimensions of the macroscopic element. If no shape can be defined for the catalyst particles, they are considered as spheres and the geometric outer dimension is the diameter of the equivalent sphere.

When the catalyst particles are assembled in an ordered way such as in fixed bed of various geometrical forms, the external volume can be calculated from the average geometric outer dimensions of the catalyst bed, using classical surface and volume formulas. The catalyst surface can be calculated from the average geometric dimensions of the outer and inner dimensions of the particles. If no shape can be defined for the catalyst particles, they are considered as spheres and the geometric outer dimension is the diameter of the equivalent sphere.

When the catalyst particles are manipulated as a whole such as in fluidised bed, entrained bed, vibrating bed, moving bed, spouted bed, bubbling bed or the like, the external volume can be calculated from the average geometric outer dimensions of the catalyst hope at rest, using classical surface and volume formulas. The catalyst surface can be calculated from the average geometric dimensions of the outer and inner dimensions of the individual catalyst particles of the hope. If no shape can be defined for the catalyst particles, they are considered as spheres and the geometric outer dimension is the diameter of the equivalent sphere.

In the process according to the invention, the catalyst particles can exhibit any form. The catalyst particle is generally in a form selected from the group consisting of rings, beads, pellets, tablets, extrudates, granules, crushed, saddled, flakes, honeycomb structures, impregnated structured packings and any mixture thereof.

When the catalyst is in the form of beads, the beads are considered as spheres and the geometric outer dimension is the diameter of the equivalent sphere.

When the catalyst is in the form of cylindrical particles (e.g. pellets, extrudates), the catalyst particles are considered as cylinders and the geometrical outer dimensions are the average particle diameter and the average particle length. The average can be geometric, arithmetic or logarithmic. The arithmetic average is for instance particularly convenient.

When the catalyst particles do not have simple geometrical form like for instance, crushed, flakes, saddles, extrudates of various forms (stars, etc.), they are considered as spheres and the geometrical outer dimensions is the diameter of the equivalent sphere.

When the catalyst particles are in the form of cylindrical rings, the catalyst particles are considered as hollow cylinders and the geometrical dimensions are the average diameters (internal and external) of the cylinders, and the average length of the cylinders.

When the catalyst is the form of a honeycomb structure with cylindrical channels, the geometrical dimensions are the average length and diameter of the channels.

Those are only a few examples on how the geometrical outer dimensions of the catalyst particles needed for calculating the external surface to volume ratio of the catalyst can be defined. The man of ordinary skill in the art will easily understand how to obtain those dimensions for any catalyst form, including the forms not disclosed hereabove.

The value of the characteristical outer dimensions of catalyst particles can be obtained by any means, for instance, by visual or microscopic measurements on individual catalyst particles followed by averaging the measure on a sufficiently large number of particles (e.g. more than 100) to be statistically reliable or from particle size distribution *via* sifting, sedimentation (natural or forced) methods or light scattering methods for instance.

In the embodiments above, the total linear velocity is understood to mean the linear velocity of the total gas feed of the reaction zone containing the catalyst.

The total linear velocity is obtained by dividing the flow of the total gas feed of the reaction zone containing the catalyst by the section of said zone.

The total gas feed can be measured by any means like for instance via orifices, venturies, nozzles, rotameters, Pitot tubes, calorimetrics, turbine, vortex, electromagnetic, Doppler, ultrasonic, thermal or Coriolis flow meters.

The section of the said reaction zone is understood to mean the average section along the length of the said reaction zone. Said reaction zone can be horizontal or vertical.

The pressure drop across the reaction zone containing the catalyst is understood to mean the dynamic pressure drop including the pressure drop corresponding to the fluid devices connected to the zone.

The pressure drop can be measured by any means like for instance differential pressure (Dp) cells, manometers such as U tube manometer, cup manometer, bourdon manometer, Piranni manometer, ionisation manometer, membrane manometer, piezo electric manometer, and any combination thereof Preferred means are selected from the group consisting of Dp cells, U tube manometer, bourdon manometer, membrane manometer, piezo electric manometer, and any combination thereof. More preferred means are selected from the group consisting of Dp cells, membrane manometer, piezo electric manometer, and any combination thereof.

The embodiments described above, which may be combined, generally allow to obtain a residence time such that a high conversion may be obtained and also, to ensure a good dispersion of the reactives inside the catalyst.

When the acetylene content of the acetylene source is high, the molar ratio of the hydrogen chloride to the acetylene introduced into the reactor is advantageously greater than or equal to about 0.5. Preferably, this ratio is greater than or equal to about 0.8. Advantageously, this molar ratio is less than or equal to about 3. Preferably, the molar ratio of the hydrogen chloride to the acetylene introduced into the reactor is less than or equal to about 1.5. Good results have been obtained when the hydrogen chloride and the acetylene are used in a molar ratio of about 0.5 to about 3.

When the acetylene content of the acetylene source is low, the molar ratio of the hydrogen chloride to the acetylene introduced into the reactor is advantageously greater than or equal to about 1000. Preferably, this ratio is greater than or equal to about 5000. Advantageously, this molar ratio is less than or equal to about 100000. Preferably, the molar ratio of the hydrogen chloride to the acetylene introduced into the reactor is less than or equal to about 50000 and even, than 20000.

The acetylene and the hydrogen chloride may be brought into contact in the reactor or, preferably, mixed prior to being introduced into the reactor.

The catalyst used according to the instant invention comprises at least one ionic liquid (a liquid which shows ionic properties at least during the process of the invention i.e. during the hydrohalogenation of the unsaturated hydrocarbon) hence comprising at least one cation and at least one anion and in one embodiment, comprising at least one non-protonated cation and at least one anion.

Ionic liquids are in principle salts in the liquid state while ordinary liquids, such as e.g. water and gasoline are predominantly made of electronically neutral molecules. Ionic liquids are advantageously made of ions. It worth noting that within the frame of the invention, imidazoles are not ionic liquids per se but in the process of the invention involving the hydrohalogenation of the unsaturated hydrocarbon, they may become ionic liquids by reaction with the hydrohalogenation reactive (HCl for instance).

It may be generally said that any salt melting without decomposition will usually yield an ionic liquid. Many salts, however, melt at high temperatures, much higher than the temperatures used in catalytic processes. For the purposes of the instant invention the term ionic liquid shall refer to a system being liquid at temperature used in the process in which the catalytic system is used.

Preferred ionic liquids for the purposes of the instant invention are those which are liquid at temperatures of 150°C or less, more preferably at temperatures of 100°C or less even more preferably at temperatures of 80°C or less. Furthermore, preferred ionic liquids are those which have a very low vapor pressure and a very low flammability and which show a good electrical conductivity.

The ionic liquid, which advantageously functions as reaction medium, has preferably a solvent capability for the reagents (unsaturated hydrocarbon and hydrohalogenation reactive like acetylene and HCl for instance) but preferably, the products (and eventually the intermediates) formed in the reaction (like VCM) are not soluble in the ionic liquid.

In the present description, the expression "at least one ionic liquid" is understood to mean one or more than one ionic liquid.

Preferably, the catalyst comprises only one ionic liquid as defined above.

In the remainder of the text, the expression "ionic liquid" used in the singular or plural should be understood as denoting one or more than one ionic liquid, except where denoted otherwise.

In the present description, the expression "at least one non-protonated cation" is understood to mean one or more than one non-protonated cation.

Preferably, the ionic liquid comprises one non-protonated cation.

In the remainder of the text, the expression "non-protonated cation" used in the singular or plural should be understood as denoting one or more than one non-protonated cation, except where denoted otherwise.

The term non-protonated cations as used herein for the purpose of the instant invention shall mean cations which do not carry free hydrogen atom(s) at the atom(s) to which the positive charge of the cation is allocated.

Advantageoulsy, the non-protonated cation is selected from

- quaternary ammonium cations which can be represented by the general formula $[NR^1R^2R^3R]^+$,
- phosphonium cations which can be represented by the general formula $[PR^1R^2R^3R]^+$, and
- cations comprising five or six-membered heterocycles which have at least one nitrogen atom, advantageously one or two nitrogen atoms.

Preferred cations comprising five or six membered heterocycles are

- imidazolium cations of the general formula (I)

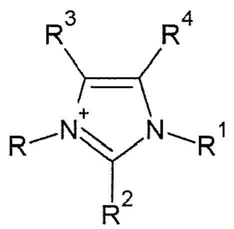

(I)

- pyridinium cations of the general formula (II), and

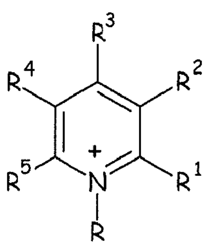

(II)

- pyrrolidinium cations of the general formula (III)

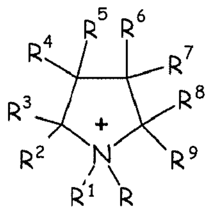

(III)

wherein radicals R and $R^1$ to $R^9$ may, independently from one another, with the proviso that the radical carried by the atom(s) to which the positive charge of the cation is allocated is not hydrogen, each be hydrogen, an optionally substituted saturated or insaturated $C_1$-$C_{18}$ alkyl group (preferably an optionally substituted saturated or insaturated $C_1$-$C_{16}$ alkyl group and more preferably an optionally substituted saturated or insaturated $C_1$-$C_{14}$ alkyl group), an optionally substituted saturated or insaturated $C_2$-$C_{18}$ alkyl group with the carbon chain interrupted by one oxygen atom or an optionally substituted $C_6$-$C_{12}$ aryl group.

Preferably, the non-protonated cation is selected from quaternary ammonium cations, phosphonium cations, imidazolium cations, pyridinium cations and pyrrolidinium cations.

More preferably, the non-protonated cation is selected from phosphonium cations, imidazolium cations, pyridinium cations and pyrrolidinium cations.

Most preferably, the non-protonated cation is selected from phosphonium cations and imidazolium cations. Especially the latter are preferred, and more specifically : dialkyl imidazolium cations, and even more preferably : 1,3 dialkyl imidazolium cations.

Examples of quaternary ammonium cations are tributylmethylammonium, butyltrimethylammonium, octyltrimethylammonium, tetramethylammonium, tetraethylammonium, tetrabutylammonium, methyltrioctylammonium, 2-hydroxyethyltrimethylammonium and diethylmethyl(2-methoxyethyl)ammonium.

Examples of phosphonium cations are triisobutylmethylphosphonium, tributylmethylphosphonium, ethyltributylphosphonium, tetrabutylphosphonium, tetraoctylphosphonium, tributyltetradecylphosphonium, trihexyltetradecylphosphonium and benzyltriphenylphosphonium.

Examples of imidazolium cations are 1,3-dimethylimidazolium, 1-ethyl-3-methylimidazolium, 1-butyl-3-methylimidazolium, 1-pentyl-3-methylimidazolium, 1-hexyl-3-methylimidazolium, 1-decyl-3-methylimidazolium, 1-dodecyl-3-methylimidazolium, 1-tetradecyl-3-methylimidazolium, 1-hexadecyl-3-methylimidazolium, 1-(2-hydroxyethyl)-3-methylimidazolium, 1-Allyl-3-methylimidazolium, 1-benzyl-3-methylimidazolium, 1-phenylpropyl-3-methylimidazolium, 1,3-diethylimidazolium, 1-butyl-3-ethylimidazolium, 1-methyl-3-propylimidazolium, 1-methyl-3-octylimidazolium, 1-methyl-3-octadecylimidazolium, 1,3-dibutyl-2-methylimidazolium, 1,3-didecyl-2-methylimidazolium, 1-(2-hydroxyethyl)-3-methylimidazolium, 1-ethyl-2,3-dimethylimidazolium, 1-propyl-2,3-dimethylimidazolium, 1-butyl-2,3-dimethylimidazolium, 1-butyl-3,4-dimethylimidazolium, 1-hexyl-2,3-dimethylimidazolium, 1-hexadecyl-2,3-dimethylimidazolium, 1,2,3-trimethylimidazolium, 1,3,4-trimethylimidazolium, 1-butyl-3-ethylimidazolium, 1,3-dibutylimidazolium, 1-methyl-3-octylimidazolium, 1-butyl-3,4,5-trimethylimidazolium and 1,3,4,5-tetramethylimidazolium.

Examples of pyridinium cations are 1-methylpyridinium, 1-ethylpyridinium, 1-propylpyridinium, 1-butylpyridinium, 1-hexylpyridinium, 1-octylpyridinium, 1,2-dimethylpyridinium, 2-ethyl-1-methylpyridinium, 1-butyl-2-methylpyridinium, 1-butyl-3-methylpyridinium, 1-butyl-4-methylpyridinium, 1-hexyl-3-methylpyridinium, 1-hexyl-4-methylpyridinium, 1-butyl-2-ethylpyridinium, 1-butyl-3-ethylpyridinium, 4-methyl-1-octylpyridinium, 1-butyl-2-ethyl-6-methylpyridinium, 2-ethyl-1,6-dimethylpyridinium, 1-butyl-3,4-dimethylpyridinium and 1-butyl-3,5-dimethylpyridinium.

Examples of pyrrolidinium cations are 1,1-dimethylpyrrolidinium, 1-ethyl-1-methylpyrrolidinium, 1-ethyl-3-methylpyrrolidinium, 1-butyl-1-methylpyrrolidinium, 1-hexyl-1-methylpyrrolidinium, 1-octyl-1-methylpyrrolidinium, 1-butyl-1-ethylpyrrolidinium and 1-methyl-1-propylpyrrolidinium.

In another embodiment, the cation may be a protonated cation like an imidazole cation, preferably an N-alkylated imidazole cation. Preferably, the N-alkylated imidazole is defined by the formula (I)

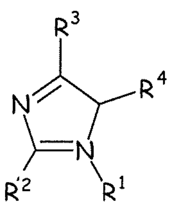

(I)

wherein radicals R1, R2, R3 and R4 may, independently from one another, each be hydrogen or an optionally substituted saturated or insaturated C1-C18 alkyl group.

More preferably, the N-alkylated imidazole is selected from 1-methylimidazole, 1-ethylimidazole, 1-butylimidazole, 1-hexylimidazole, 1-octylimidazole, 1-decylimidazole, 1-methyl-2-octylimidazole, 1-ethyl-2-methylimidazole, 1-butyl-2-methylimidazole, 1-hexyl-2-methylimidazole and 1-decyl-2-methylimidazole. N-alkylated imidazoles selected from 1-methylimidazole, 1-ethylimidazole and 1-butylimidazole are even more preferred.

In the present description, the expression "at least one anion" is understood to mean one or more than one anion.

Preferably, the ionic liquid comprises one anion.

In the remainder of the text, the expression "anion" used in the singular or plural should be understood as denoting one or more than one anion, except where denoted otherwise.

As anions, it is in principle possible to use all anions. The anion is preferably selected from :

the group consisting of halides and halogen-containing anions and so called pseudo-halides,
the group consisting of sulfates, sulfites and sulfonates,
the group consisting of phosphates,
the group consisting of phosphonates and phosphinates,
the group consisting of phosphites,
the group consisting of phosphonites and phosphinites,
the group consisting of carboxylic acids,
the group consisting of borates,
the group consisting of boronates,
the group consisting of carbonates and carbonic esters,
the group consisting of silicates and silicic esters,
the group consisting of alkylsilane and arylsilane salts,
the group consisting of carboximides, bis(sulfonyl)imides and sulfonylimides,
the group consisting of alkoxides and aryloxides.

The anion(s) are preferably chosen among the following ones : chloride, bromide, iodide, triflate (trifluoromethanesulfonate), tosylate, tetrafluoroethylsulfonate, bis-trifluoromethylsulfonylimide, tetrachloroferrate, tetrafluoroborate, tetrafluorophosphate and hexafluorophosphate.

The most preferred IL are imidazolium halogenated salts and more specifically : chlorides. Especially BMIMCl or 1-Butyl-3-methylimidazolium chloride is suitable because readily available commercially.

Methods for the manufacture of suitable ionic liquids are known to the skilled man and thus a detailed description is not necessary here, especially since some of them and especially BMIMCl are commercially available.

The catalyst in accordance with the instant invention comprises a solid carrier or support such as a silica, alumina, silica alumina, silicon carbide, cordierite, mullite or activated carbon (to name only a few suitable support materials), up to the limit of the pore volume and the available surface of the support. Silica gives good results in the frame of the invention, especially for the hydrochlorination of acetylene using BMIMCl as IL and Pd as metal.

In a one embodiment of the invention, the support of the catalyst has a BET surface of at least 0.1 $m^2/g$, preferably of at least 3.0 $m^2/g$ and even more preferably of at least 10 $m^2/g$. According to this aspect of the invention, BET surface is preferably determined as set forth in the Examples, noting that the accuracy may be about 5 %.

In another embodiment of the invention, the support of the catalyst has a pore volume (PV) of at least 0.02 mL/g, more preferably at least 0.2 and most preferably at least 0.4 mL/g. According to the invention, the pore volume is measured according to standard ISO 15901-3:2007.

The 2 embodiments above (based on BET surface and PV characteristics), which may eventually be combined, generally allow effectively impregnating said support with the IL (see preparation method described below) so that a highly productive catalyst may be obtained.

It should further be noted that any of the above described embodiments (either based on process characteristics (S/V of the catalyst, linear gas velocity or pressure drop) or on support characteristics (BET surface or PV)) may be used alone, or in combination with at least one of the others. At least one embodiment of each category may be combined with at least one embodiment of the other category. All embodiments may also be combined.

The catalyst of the invention also comprises a metal. The metal can be any metal. The metal is advantageously chosen from Pd, Pt, Au, Hg, Ru, Os, Ru, Rh and Ir. Preferably, the metal is chosen from Pd, Pt, Au, Hg, Ru and Os.

While good results have been obtained when the metal is chosen among the ones cited above, very good results have been obtained when the metal is chosen from Pd, Ru, Au and Os, particularly very good results have been obtained when the metal is chosen from Pd, Ru and Au and more particularly very good results have been obtained when the metal is chosen from Pd and Ru. The most interesting results have been obtained when metal is Pd.

The metal content of the catalyst according to the invention, is preferably equal to or higher than 0.25 wt %

(based on the total weight of catalyst), preferably than 0.5 wt %. It is generally equal to or lower than 10 wt %, preferably than 5 wt %.

Generally, the catalytic system in accordance with the invention is prepared by dissolving the desired amount of at least one metal compound in the IL, melted if required. Then, preferably, in order to avoid gluing (agglomeration) of the mixture, a solvent is added, which may be water, acetone or the like. Water gives good results in practice and is more environmental friendly. Alternatively, the desired amount of at least one metal compound is dissolved in the solvent and the IL, melted if required, is added afterwards. After that, the mixture is contacted with the support. In the preferred case, where a solvent is utilized, the excess of solvent is removed by evaporation techniques. The support and the whole may eventually be calcined.

Alternatively, the catalyst in accordance with the invention is prepared by first depositing at least one metal compound on the support, which may then be calcined. Afterwards, said support is impregnated with the IL, again melted if required (i.e. the temperature of the IL should be adapted so that it is effectively liquid).

The expression "at least one metal compound" as used herein includes single metal compounds of one metal as well as mixtures of different compounds of the same metal or mixtures of compounds of different metals or compounds comprising two metals as defined hereinbefore.

Preferably, the catalyst is obtained from one compound of at least one metal and more preferably from one compound of one metal.

The metal compound may be of any nature ; however, it is generally a salt, more preferably a halide and even more preferably, a chloride.

Among the chloride-based compounds of palladium (II), mention may be made of palladium (II) chloride and the palladochlorides of alkali metals or of alkaline-earth metals, such as for example $Na_2(PdCl_4)$, $K_2(PdCl_4)$, $Li_2(PdCl_4)$ and $(NH_4)_2(PdCl_4)$. Palladium (II) chloride gives good results.

Although the corrosion problem is strongly reduced with the process of the invention, some materials are nevertheless corroded when subjected to the process of the invention for a long time. Hence, in a preferred embodiment, the process of the invention is at least partly carried out in apparatus made from or covered with materials which are resistant to halogenated acids in dissociated form and which are chosen from metals, polymers, ceramics, refractory materials, (impregnated) graphite and enamel (see above and in co-pending application PCT/EP2012/052911 for more details on these materials).

By the terms "at least partly carried out" is meant that at least that part of the apparatus where the reaction is carried out, and which is in contact with the reaction medium, is made of one of the above mentioned corrosion resistant materials.

By the terms "resistant to halogenated acids (HCl) in dissociated form" is meant that the concerned materials can be used for said part of the apparatus during a normal industrial life time (several years, typically at least 10 years) without needing to be replaced.

The examples hereafter are intended to illustrate the invention without however limiting the scope thereof.

General experimental procedure used for the examples:

Catalyst synthesis :

BMIMCl was first melted during 5 min at 100°C. Then, $PdCl_2$ was added under stirring during 45 min. Water was then added still under stirring for an additional 10 min. The support was then added to the solution, which was afterwards evaporated at 90°C and under 150 mbar vacuum in a rotovapor in order to remove water. The evaporation procedure lasted at least 5 h for the catalysts with an IL weight content less than or equal to 30 %. For the catalysts with higher IL contents, the evaporation procedure was extended at least to 10 h in total. The obtained catalysts were then dried overnight in an oven at 105°C.

*Support characterization*

BET surface and meso-pore volume were measured as follows : between 0.4 and 0.6 g of desired sample were firstly degassed at 200°C for 16h. Results were then obtained with nitrogen using a Micromeritics ASAP 2020 V3.05 H using a multi-point evaluation for P/P0 between 0.01 and 0.2. Macro-pore volume was measured as follows : between 0.4 and 0.6 g of desired sample were firstly degassed at 200°C for 16h. Results were then obtained with mercury using a Micromeritics Autopore IV 9500 V1.09 with a pressure range between 0.0035 and 400 MPa.

*Hydrochlorination reactions :*

Hydrochlorination reactions were performed in a glass reactor having a diameter of 10 mm equipped at the bottom with a sintered glass disk to obtain a good dispersion of the gaseous reactants composed of acetylene and

hydrochloric acid.

The catalyst particles (having a mean particle size of about 0.15 mm, so a S/V ratio of 40000) were arranged as a fixed bed lying on the bottom of the reactor.

The thermal control of the system was obtained by a double wall reactor with a thermal oil regulation to maintain the desired temperature controlled by a thermowell placed in the reactor itself.

*Tests & Results*

First, the unsupported IL-Pd system was tested. PdC12 was dissolved in 15 g of melted BMIMCl in order to obtain a Pd molar concentration of 22.6 mM. Hydrochlorination at 150°C with a gaseous flow rate of 5 Nl/hour for C2H2 and 6 Nl/hour for HCl lead to a C2H2 conversion of 47 %, and a selectivity above 99.8 %, leading to a productivity (expressed in g VCM/l catalyst/h) of 400.

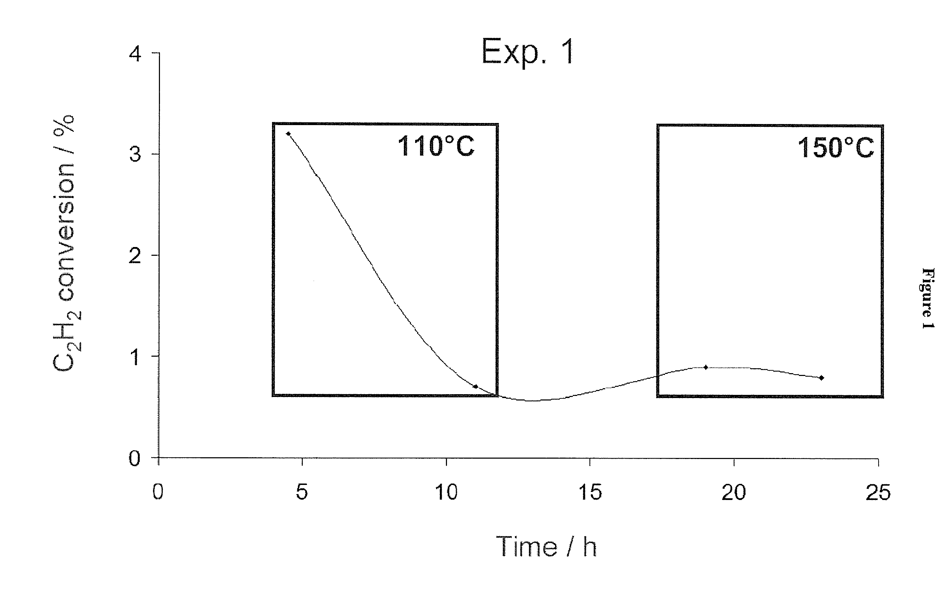

The other experiments were made using a support, the nature of said support, the experimental conditions and the results thereof being summarized in Table 1 and Figures 1 to 3 attached.

First, the raw SiO2 support loaded with Pd only (0.1396 g of $PdCl_2$) were dissolved in approximately 30 mL of water under stirring at 80°C. Few drops of HCl 37 v/v % were added to complete the $PdCl_2$ dissolution. After the complete dissolution of the $PdCl_2$, 4 g of $SiO_2$ were added under stirring. The slurry was stirred for 30 min and then the water was evaporated at the rotovapor at 90°C and 150 mbar for at least 4 h. The solid obtained was dried overnight at 105°C) and then was tested at 110°C and 150°C in the conditions indicated in Table 1 (Exp. 1). As can be seen from Figure 1, the C2H2 conversion (in %) obtained was negligible (from about 1 to 3 % only).

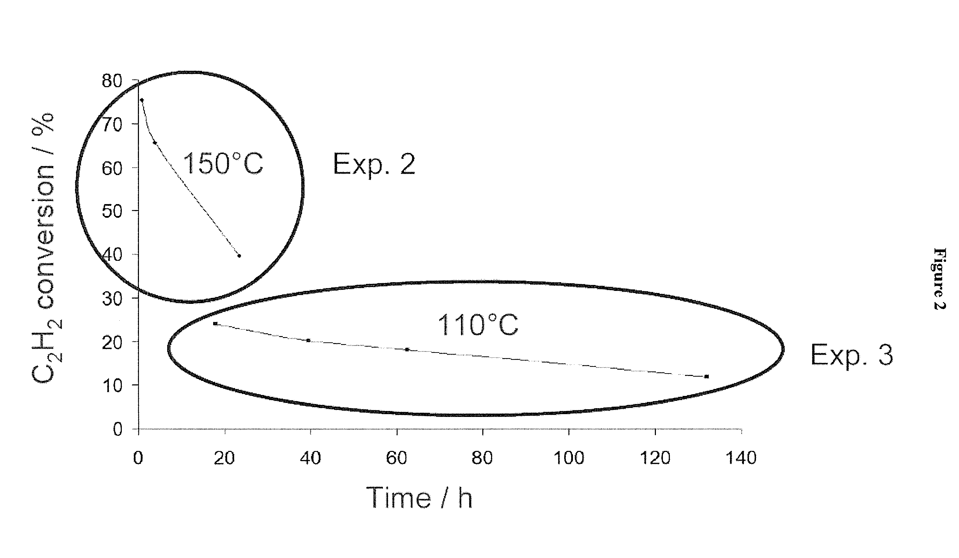

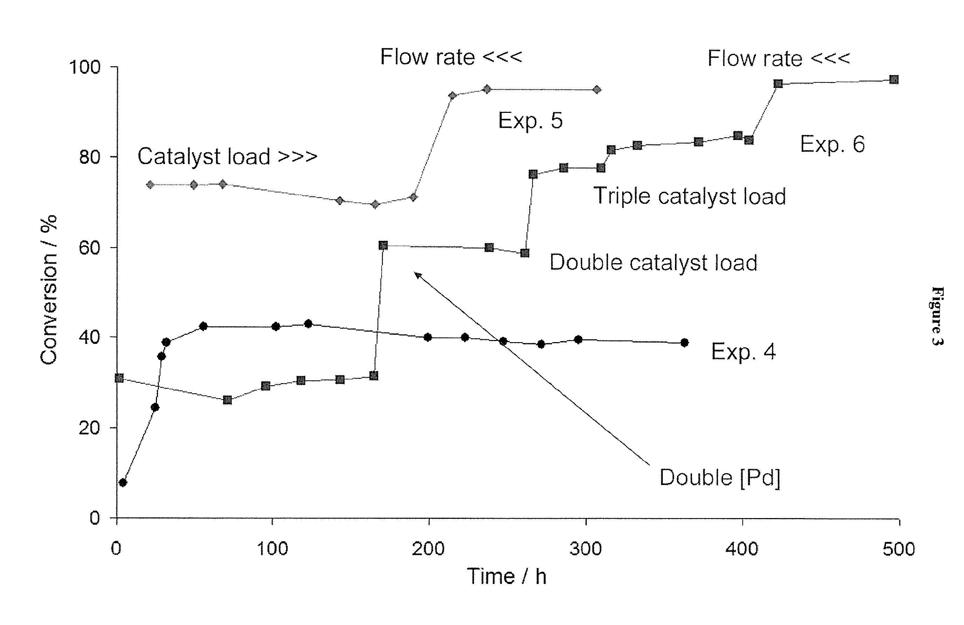

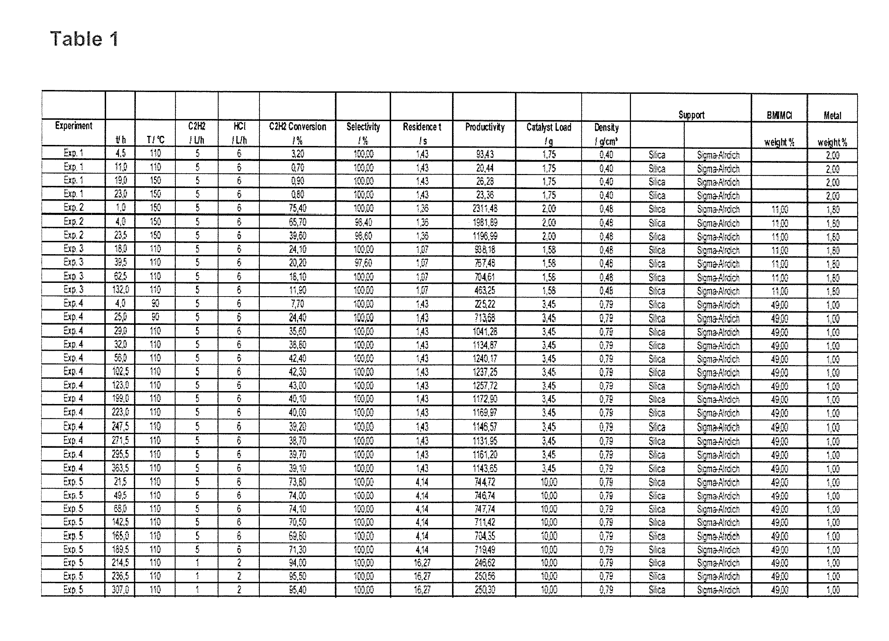

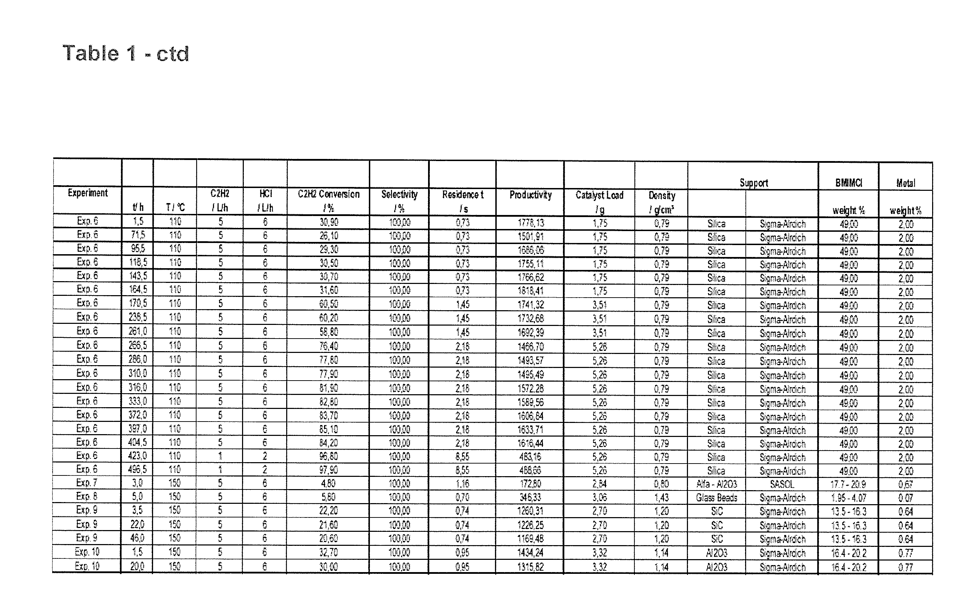

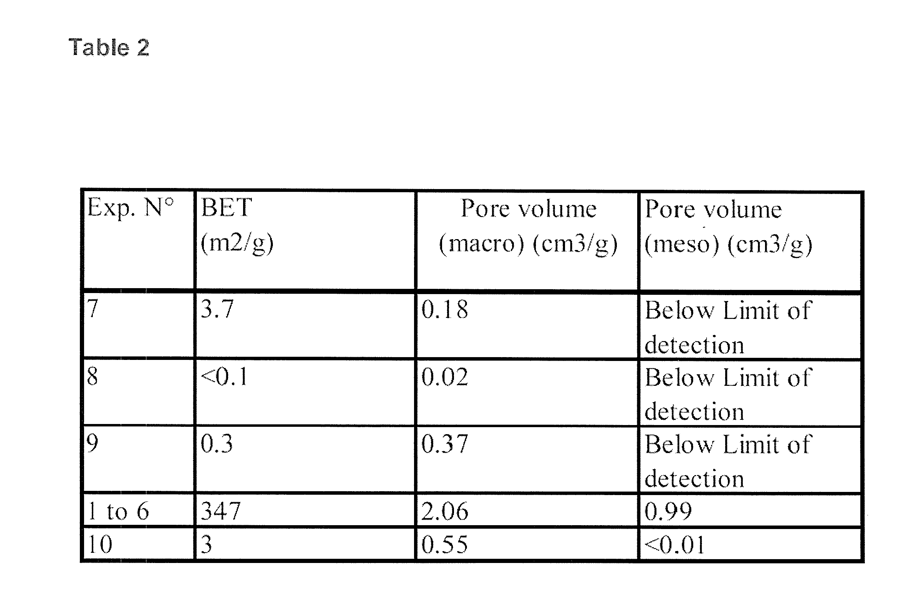

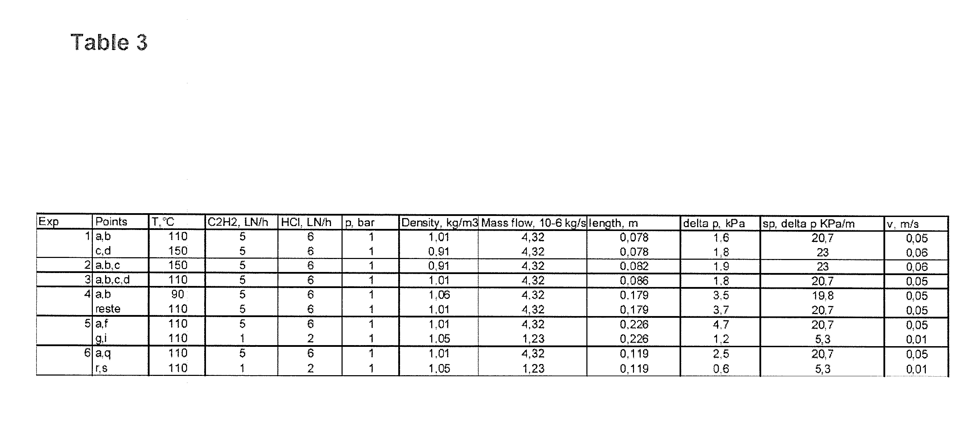

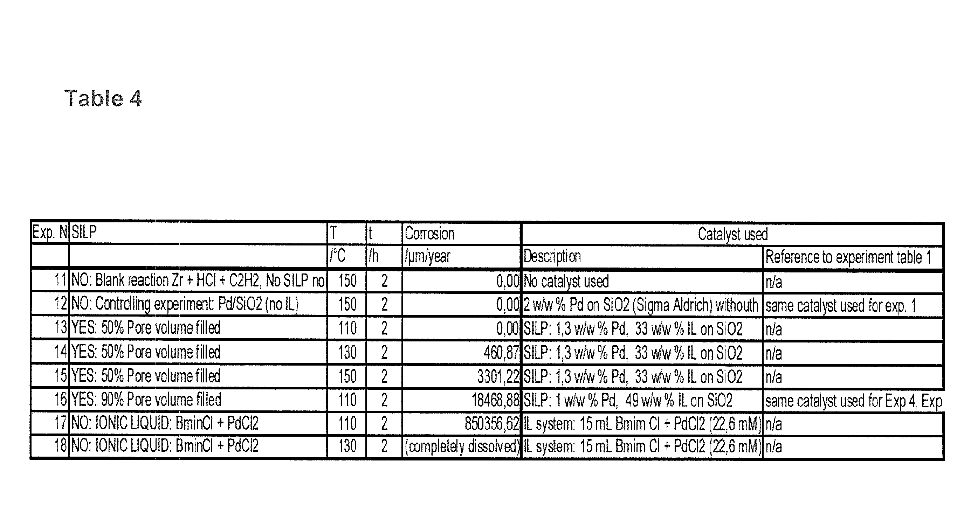

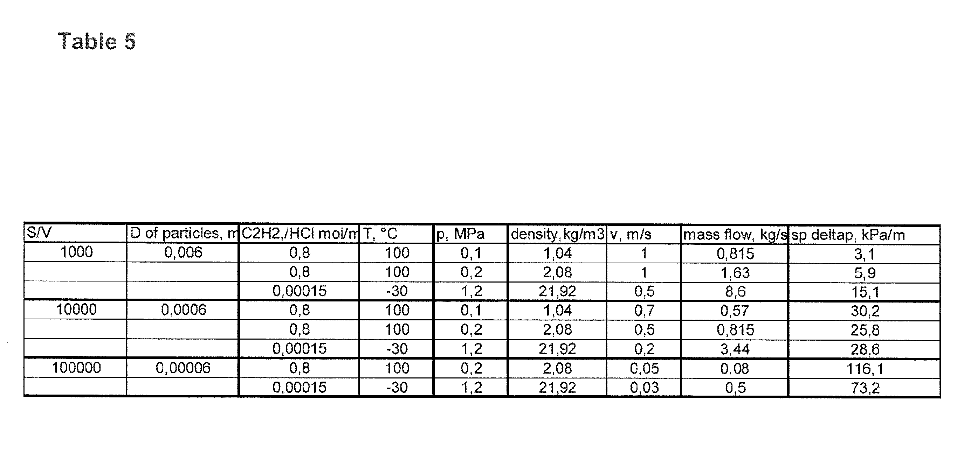

Second, the same support was loaded with different amounts of IL according to the above synthesis procedure and using the conditions of Table 1. The results are shown in said Table 1 and in Figures 2 and 3 attached.

Figure 2 relates to experiments 2 and 3 (Exp. 2 and Exp. 3) of Table 1 using a SILP with 10 % pore volume filled with IL, respectively at a temperature of:

- 150°C (Exp. 2), where the initial conversion was bout 75 %, with a selectivity of about 99 %. However, deactivation occurred after couple of hours (decrease in conversion down to about 60 %). The associated initial productivity was about 2300.
- 110°C (Exp. 3), where the conversion rate was, as shown, less high (about 20 %) but more stable.

Analysis on exhausted catalyst proved the presence of high amounts of carbonaceous species for 150°C reaction. Also, presence of black 'stuff in the glass ball after reactor was detected.

Figure 3 relates to experiments 4 to 6 of Table 1 using a SILP with 90 % pore volume filled with IL at a temperature of 110°C, temperature which could not be increased due to gluing side effects. For Exp. 4, the conversion was bout 40 %, with a selectivity of more than 99.8 %, leading to a productivity of about 1150. Analysis of the recovered catalyst did not shown any presence of coke.

As can be seen from this last figure, increasing the load of catalyst in the reactor and reducing the flows (i.e. increasing the residence time) allows to reach almost 100 % conversion. However, increasing the Pd content does not seem to have a positive effect.

Table 1, in combination with Table 2 attached, also shows that with supports having a low BET surface and/or low pore volume, the C2H2 conversions reached are much lower (compare Exp. 2 with Exp. 7-10). Also, for the catalysts obtained with these supports, since their porosity was low, all the BMIMCl used during the preparation of the catalysts could not be absorbed. Hence, their actual BMIMCl content was measured with an experimental error reflected in the ranges indicated. For the other catalysts, the measurements confirmed all the BMIMCl was actually absorbed.

Finally, Table 3 shows that during Experiments 1 to 6, the pressure drop within the fixed bed catalyst was within the preferred range set forth above (namely : below 50 kPa/m) and the same applies for the linear velocity of the gases (namely : above 0.005 m/s and below 20 m/s). In this table, the "points" (a, b, c and d for Exp. 1 for instance) are the results given for each time (line) in Table 1 (again for Exp.1 for instance : respectively after 4.5, 11, 19 and 23 h).

*Corrosion tests*

First, a plate of 1.5+/- 0.2 mm of zirconium was inserted inside a reactor. The catalyst was then added in a way that allowed the coverage of at least 75 % of the total surface of the plate. The reaction was then started under the above mentioned C2H2 and HCl flow rates at a given temperature and stopped after the desired time. The plate was recovered and then sent for analysis. The results and conditions are shown in Table 4 attached. Each corrosion experiment was performed using an amount of fresh catalyst. These results where obtained according to ASTM G1, 1999 version.

*Industrial simulation tests*

Estimated pressure drops in a fixed bed catalyst of 1m diameter operated in several conditions are provided in Table 5 attached.

This table shows that if a S/V ratio outside the preferred range set forth above for fixed bed catalysts (namely below 6 $10^4$) is chosen, the resulting pressure drop is also outside the preferred range set forth above for fixed bed catalysts (namely below 50 kPa/m).

## Claims

1. Process for the hydrohalogenation of an unsaturated hydrocarbon using a catalyst comprising at least one ionic liquid (IL) and a metal, according to which said IL and metal are supported on a solid carrier, said process being a continuous industrial process wherein the overall unsaturated hydrocarbon conversion is of at least 60 % during at least 500 hours.

2. Process according to the preceding claim, wherein the overall selectivity of the catalyst is of at least 90 % during at least 500 hours.

3. Process according to any of the preceding claims, wherein during at least 500 hours :

   - when the unsaturated hydrocarbon source has an unsaturated hydrocarbon content equal to or above 10 %, the productivity of the catalyst is of at least 100 expressed in g of hydrohalogenated unsaturated hydrocarbon per liter of catalyst and per hour
   - when the unsaturated hydrocarbon content of the unsaturated hydrocarbon source is below 10 %, the productivity of the catalyst is of at least 1.

4. Process according to any of the preceding claims, wherein the unsaturated hydrocarbon is acetylene and the hydrohalogenation is hydrochlorination so as to produce vinyl chloride monomer (VCM).

5. Process according to the preceding claim, said process being carried out after a pyrolysis step of 1,2-dichoroethane (DCE) in a process to produce DCE and VCM from ethylene.

6. Process according to the preceding claim, wherein the catalyst is of the honeycomb type and has large channels so as to avoid clogging of said channels by tars.

7. Process according to any of the preceding claims, wherein :

   - when the unsaturated hydrocarbon source has an unsaturated hydrocarbon content equal to or above 10 %, the hydrochlorination reaction is carried out at a temperature of about 40°C to about 200°C
   - when the unsaturated hydrocarbon content of the unsaturated hydrocarbon source is below 10 %, the hydrochlorination reaction is carried out at a temperature between -30°C and about 200°C.

8. Process according to any of the preceding claims, wherein :

   - when the unsaturated hydrocarbon source has an unsaturated hydrocarbon content equal to or above 10 %, the hydrochlorination reaction is carried out at a pressure below 10 MPa
   - when the unsaturated hydrocarbon content of the unsaturated hydrocarbon source is below 10 %, the hydrochlorination reaction is carried out at a pressure below 5 MPa.

9. Process according to any of the preceding claims, wherein the reaction is carried out in a fixed bed and/or in a preassembled structure, wherein the external surface to volume ratio (S/V) of the catalyst is lower than or equal to 6 $10^4$ $m^{-1}$ but higher than 10 $m^{-1}$, said process comprising feeding continuously a reaction zone comprising the catalyst with at least the unsaturated hydrocarbon and the hydrohalogenation reactive, both in gaseous form, at a total linear velocity higher than or equal to 0.005 m/s but lower than or equal to 20 m/s and with a pressure drop across the reaction zone which is lower than or equal to 50 kPa/m.

10. Process according to any of claims 1 to 8, wherein the reaction is carried out in a fluidised flow, wherein the external

surface to volume ratio (S/V) of the catalyst is lower than or equal to $10^5$ $m^{-1}$ but higher than 100 $m^{-1}$, said process comprising feeding continuously a reaction zone comprising the catalyst with at least the unsaturated hydrocarbon and the hydrohalogenation reactive, both in gaseous form, at a total linear velocity higher than or equal to 0.15 m/s but lower than or equal to 6 m/s and with a pressure drop across the reaction zone which is lower than or equal to 100 kPa/m.

**11.** Process according to any of claims 1 to 8, wherein the reaction is carried out in an entrained flow, wherein the external surface to volume ratio (S/V) of the catalyst is lower than or equal to 2 $10^6$ $m^{-1}$ but higher than 100 $m^{-1}$', said process comprising feeding continuously a reaction zone comprising the catalyst with at least the unsaturated hydrocarbon and the hydrohalogenation reactive, both in gaseous form, at a total linear velocity higher than or equal to 0.25 m/s but lower than or equal to 20 m/s and with a pressure drop across the reaction zone which is lower than or equal to 50 kPa/m.

**12.** Process according to any of the preceding claims, wherein :

- when the unsaturated hydrocarbon source has an unsaturated hydrocarbon content equal to or above 10 %, the molar ratio of the hydrogen chloride to the acetylene is greater than or equal to about 0.5 but less than or equal to 1.5
- when the unsaturated hydrocarbon content of the unsaturated hydrocarbon source is below 10 %, the molar ratio of the hydrogen chloride to the acetylene is greater than or equal to about 1000 but less than or equal to 100000.

**13.** Process according to any of the preceding claims, wherein the IL is 1-Butyl-3-methylimidazolium chloride (BMIMCl).

**14.** Process according to any of the preceding claims, wherein the solid carrier of the catalyst has a BET surface of at least 0.1 $m^2$/g.

**15.** Process according to any of the preceding claims, wherein the solid carrier of the catalyst has a pore volume (PV) of at least 0.02 $cm^3$/g.

**Figure 1**

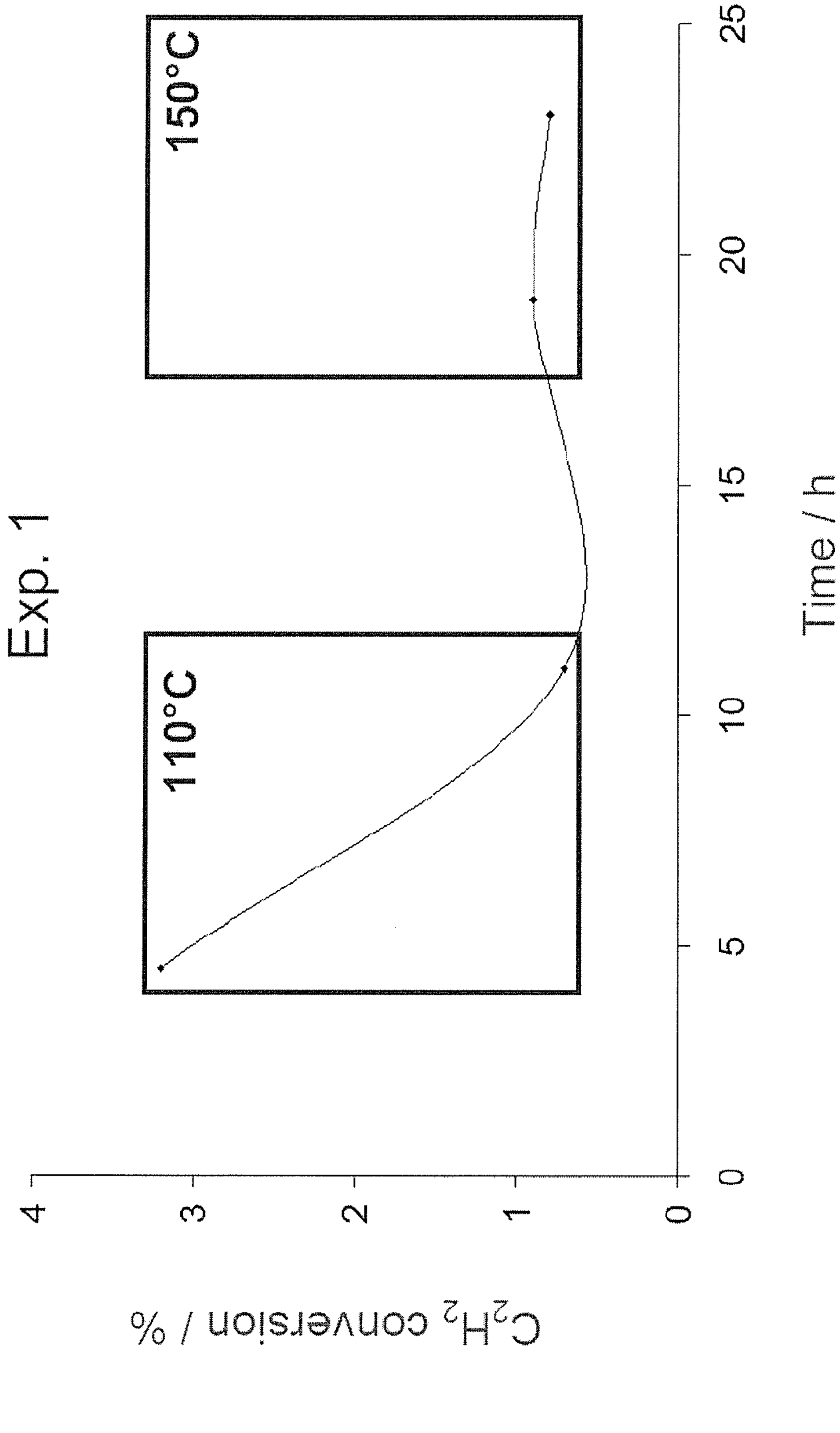

**Figure 2**

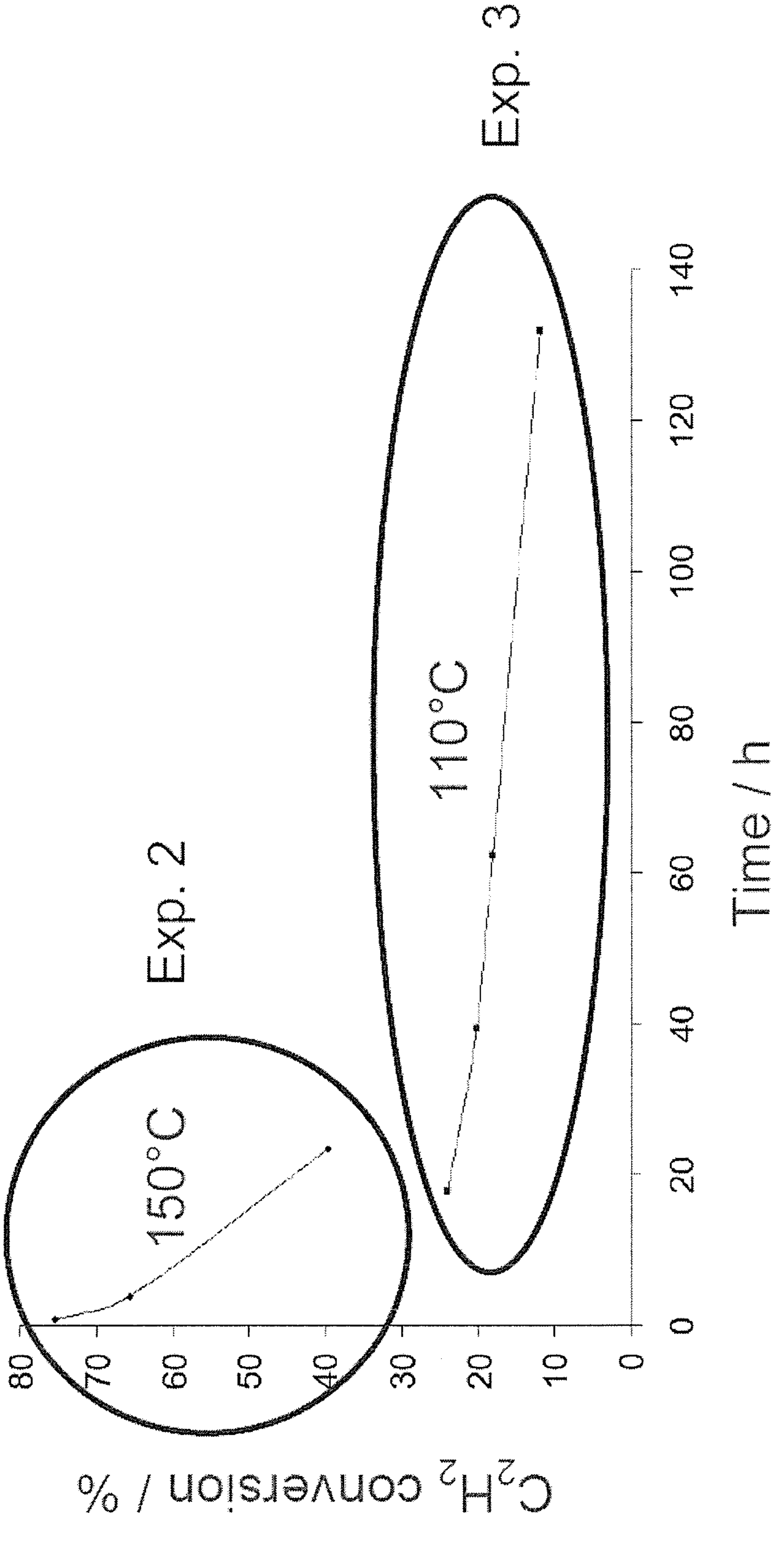
Exp. 2
Exp. 3
150°C
110°C
Time / h
$C_2H_2$ conversion / %
0
20
40
60
80
100
120
140
0
10
20
30
40
50
60
70
80

Figure 3

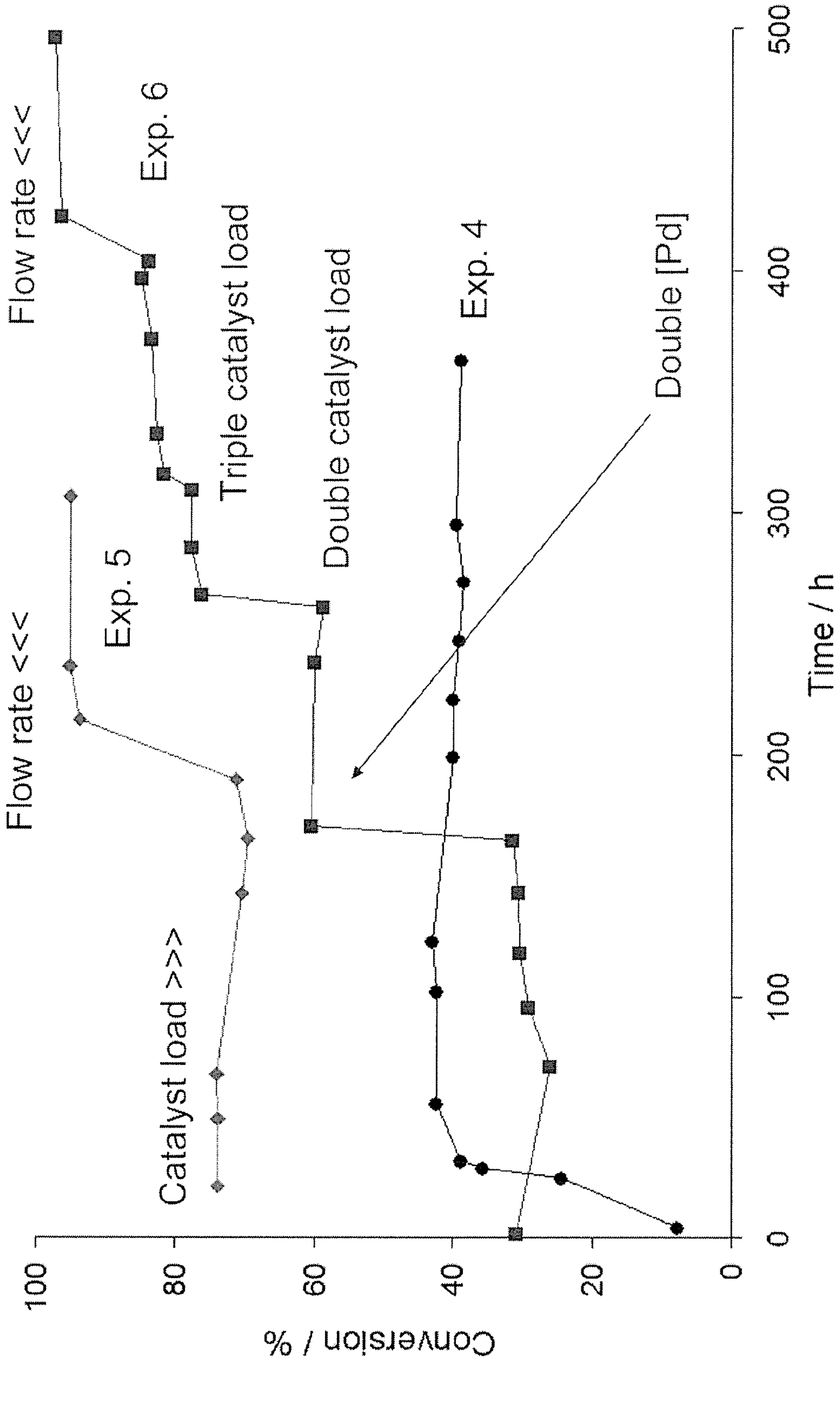
Flow rate <<<
Exp. 6
Triple catalyst load
Double catalyst load
Exp. 4
Double [Pd]
Flow rate <<<
Exp. 5
Catalyst load >>>
Conversion / %
Time / h
0
20
40
60
80
100
0
100
200
300
400
500

Table 1

| Experiment | t/ h | T / °C | C2H2 / L/h | HCl / L/h | C2H2 Conversion / % | Selectivity / % | Residence t / s | Productivity | Catalyst Load / g | Density / g/cm³ | Support | | BMIMCl weight % | Metal weight % |
|---|---|---|---|---|---|---|---|---|---|---|---|---|---|---|
| Exp. 1 | 4,5 | 110 | 5 | 6 | 3,20 | 100,00 | 1,43 | 93,43 | 1,75 | 0,40 | Silica | Sigma-Alrdich | | 2,00 |
| Exp. 1 | 11,0 | 110 | 5 | 6 | 0,70 | 100,00 | 1,43 | 20,44 | 1,75 | 0,40 | Silica | Sigma-Alrdich | | 2,00 |
| Exp. 1 | 19,0 | 150 | 5 | 6 | 0,90 | 100,00 | 1,43 | 26,28 | 1,75 | 0,40 | Silica | Sigma-Alrdich | | 2,00 |
| Exp. 1 | 23,0 | 150 | 5 | 6 | 0,80 | 100,00 | 1,43 | 23,36 | 1,75 | 0,40 | Silica | Sigma-Alrdich | | 2,00 |
| Exp. 2 | 1,0 | 150 | 5 | 6 | 75,40 | 100,00 | 1,36 | 2311,48 | 2,00 | 0,48 | Silica | Sigma-Alrdich | 11,00 | 1,80 |
| Exp. 2 | 4,0 | 150 | 5 | 6 | 65,70 | 96,40 | 1,36 | 1981,89 | 2,00 | 0,48 | Silica | Sigma-Alrdich | 11,00 | 1,80 |
| Exp. 2 | 23,5 | 150 | 5 | 6 | 39,60 | 98,60 | 1,36 | 1196,99 | 2,00 | 0,48 | Silica | Sigma-Alrdich | 11,00 | 1,80 |
| Exp. 3 | 18,0 | 110 | 5 | 6 | 24,10 | 100,00 | 1,07 | 938,18 | 1,58 | 0,48 | Silica | Sigma-Alrdich | 11,00 | 1,80 |
| Exp. 3 | 39,5 | 110 | 5 | 6 | 20,20 | 97,60 | 1,07 | 757,48 | 1,58 | 0,48 | Silica | Sigma-Alrdich | 11,00 | 1,80 |
| Exp. 3 | 62,5 | 110 | 5 | 6 | 18,10 | 100,00 | 1,07 | 704,61 | 1,58 | 0,48 | Silica | Sigma-Alrdich | 11,00 | 1,80 |
| Exp. 3 | 132,0 | 110 | 5 | 6 | 11,90 | 100,00 | 1,07 | 463,25 | 1,58 | 0,48 | Silica | Sigma-Alrdich | 11,00 | 1,80 |
| Exp. 4 | 4,0 | 90 | 5 | 6 | 7,70 | 100,00 | 1,43 | 225,22 | 3,45 | 0,79 | Silica | Sigma-Alrdich | 49,00 | 1,00 |
| Exp. 4 | 25,0 | 90 | 5 | 6 | 24,40 | 100,00 | 1,43 | 713,68 | 3,45 | 0,79 | Silica | Sigma-Alrdich | 49,00 | 1,00 |
| Exp. 4 | 29,0 | 110 | 5 | 6 | 35,60 | 100,00 | 1,43 | 1041,28 | 3,45 | 0,79 | Silica | Sigma-Alrdich | 49,00 | 1,00 |
| Exp. 4 | 32,0 | 110 | 5 | 6 | 38,80 | 100,00 | 1,43 | 1134,87 | 3,45 | 0,79 | Silica | Sigma-Alrdich | 49,00 | 1,00 |
| Exp. 4 | 56,0 | 110 | 5 | 6 | 42,40 | 100,00 | 1,43 | 1240,17 | 3,45 | 0,79 | Silica | Sigma-Alrdich | 49,00 | 1,00 |
| Exp. 4 | 102,5 | 110 | 5 | 6 | 42,30 | 100,00 | 1,43 | 1237,25 | 3,45 | 0,79 | Silica | Sigma-Alrdich | 49,00 | 1,00 |
| Exp. 4 | 123,0 | 110 | 5 | 6 | 43,00 | 100,00 | 1,43 | 1257,72 | 3,45 | 0,79 | Silica | Sigma-Alrdich | 49,00 | 1,00 |
| Exp. 4 | 199,0 | 110 | 5 | 6 | 40,10 | 100,00 | 1,43 | 1172,90 | 3,45 | 0,79 | Silica | Sigma-Alrdich | 49,00 | 1,00 |
| Exp. 4 | 223,0 | 110 | 5 | 6 | 40,00 | 100,00 | 1,43 | 1169,97 | 3,45 | 0,79 | Silica | Sigma-Alrdich | 49,00 | 1,00 |
| Exp. 4 | 247,5 | 110 | 5 | 6 | 39,20 | 100,00 | 1,43 | 1146,57 | 3,45 | 0,79 | Silica | Sigma-Alrdich | 49,00 | 1,00 |
| Exp. 4 | 271,5 | 110 | 5 | 6 | 38,70 | 100,00 | 1,43 | 1131,95 | 3,45 | 0,79 | Silica | Sigma-Alrdich | 49,00 | 1,00 |
| Exp. 4 | 295,5 | 110 | 5 | 6 | 39,70 | 100,00 | 1,43 | 1161,20 | 3,45 | 0,79 | Silica | Sigma-Alrdich | 49,00 | 1,00 |
| Exp. 4 | 363,5 | 110 | 5 | 6 | 39,10 | 100,00 | 1,43 | 1143,65 | 3,45 | 0,79 | Silica | Sigma-Alrdich | 49,00 | 1,00 |
| Exp. 5 | 21,5 | 110 | 5 | 6 | 73,80 | 100,00 | 4,14 | 744,72 | 10,00 | 0,79 | Silica | Sigma-Alrdich | 49,00 | 1,00 |
| Exp. 5 | 49,5 | 110 | 5 | 6 | 74,00 | 100,00 | 4,14 | 746,74 | 10,00 | 0,79 | Silica | Sigma-Alrdich | 49,00 | 1,00 |
| Exp. 5 | 68,0 | 110 | 5 | 6 | 74,10 | 100,00 | 4,14 | 747,74 | 10,00 | 0,79 | Silica | Sigma-Alrdich | 49,00 | 1,00 |
| Exp. 5 | 142,5 | 110 | 5 | 6 | 70,50 | 100,00 | 4,14 | 711,42 | 10,00 | 0,79 | Silica | Sigma-Alrdich | 49,00 | 1,00 |
| Exp. 5 | 165,0 | 110 | 5 | 6 | 69,80 | 100,00 | 4,14 | 704,35 | 10,00 | 0,79 | Silica | Sigma-Alrdich | 49,00 | 1,00 |
| Exp. 5 | 169,5 | 110 | 5 | 6 | 71,30 | 100,00 | 4,14 | 719,49 | 10,00 | 0,79 | Silica | Sigma-Alrdich | 49,00 | 1,00 |
| Exp. 5 | 214,5 | 110 | 1 | 2 | 94,00 | 100,00 | 16,27 | 246,62 | 10,00 | 0,79 | Silica | Sigma-Alrdich | 49,00 | 1,00 |
| Exp. 5 | 236,5 | 110 | 1 | 2 | 95,50 | 100,00 | 16,27 | 250,56 | 10,00 | 0,79 | Silica | Sigma-Alrdich | 49,00 | 1,00 |
| Exp. 5 | 307,0 | 110 | 1 | 2 | 95,40 | 100,00 | 16,27 | 250,30 | 10,00 | 0,79 | Silica | Sigma-Alrdich | 49,00 | 1,00 |

# Table 1 - ctd

| Experiment | t/ h | T / °C | C2H2 / L/h | HCl / L/h | C2H2 Conversion / % | Selectivity / % | Residence t / s | Productivity | Catalyst Load / g | Density / g/cm³ | Support | | BMIMCl weight % | Metal weight % |
|---|---|---|---|---|---|---|---|---|---|---|---|---|---|---|
| Exp. 6 | 1,5 | 110 | 5 | 6 | 30,90 | 100,00 | 0,73 | 1778,13 | 1,75 | 0,79 | Silica | Sigma-Alrdich | 49,00 | 2,00 |
| Exp. 6 | 71,5 | 110 | 5 | 6 | 26,10 | 100,00 | 0,73 | 1501,91 | 1,75 | 0,79 | Silica | Sigma-Alrdich | 49,00 | 2,00 |
| Exp. 6 | 95,5 | 110 | 5 | 6 | 29,30 | 100,00 | 0,73 | 1686,06 | 1,75 | 0,79 | Silica | Sigma-Alrdich | 49,00 | 2,00 |
| Exp. 6 | 118,5 | 110 | 5 | 6 | 30,50 | 100,00 | 0,73 | 1755,11 | 1,75 | 0,79 | Silica | Sigma-Alrdich | 49,00 | 2,00 |
| Exp. 6 | 143,5 | 110 | 5 | 6 | 30,70 | 100,00 | 0,73 | 1766,62 | 1,75 | 0,79 | Silica | Sigma-Alrdich | 49,00 | 2,00 |
| Exp. 6 | 164,5 | 110 | 5 | 6 | 31,60 | 100,00 | 0,73 | 1818,41 | 1,75 | 0,79 | Silica | Sigma-Alrdich | 49,00 | 2,00 |
| Exp. 6 | 170,5 | 110 | 5 | 6 | 60,50 | 100,00 | 1,45 | 1741,32 | 3,51 | 0,79 | Silica | Sigma-Alrdich | 49,00 | 2,00 |
| Exp. 6 | 238,5 | 110 | 5 | 6 | 60,20 | 100,00 | 1,45 | 1732,68 | 3,51 | 0,79 | Silica | Sigma-Alrdich | 49,00 | 2,00 |
| Exp. 6 | 261,0 | 110 | 5 | 6 | 58,80 | 100,00 | 1,45 | 1692,39 | 3,51 | 0,79 | Silica | Sigma-Alrdich | 49,00 | 2,00 |
| Exp. 6 | 266,5 | 110 | 5 | 6 | 76,40 | 100,00 | 2,18 | 1466,70 | 5,26 | 0,79 | Silica | Sigma-Alrdich | 49,00 | 2,00 |
| Exp. 6 | 286,0 | 110 | 5 | 6 | 77,80 | 100,00 | 2,18 | 1493,57 | 5,26 | 0,79 | Silica | Sigma-Alrdich | 49,00 | 2,00 |
| Exp. 6 | 310,0 | 110 | 5 | 6 | 77,90 | 100,00 | 2,18 | 1495,49 | 5,26 | 0,79 | Silica | Sigma-Alrdich | 49,00 | 2,00 |
| Exp. 6 | 316,0 | 110 | 5 | 6 | 81,90 | 100,00 | 2,18 | 1572,28 | 5,26 | 0,79 | Silica | Sigma-Alrdich | 49,00 | 2,00 |
| Exp. 6 | 333,0 | 110 | 5 | 6 | 82,80 | 100,00 | 2,18 | 1589,56 | 5,26 | 0,79 | Silica | Sigma-Alrdich | 49,00 | 2,00 |
| Exp. 6 | 372,0 | 110 | 5 | 6 | 83,70 | 100,00 | 2,18 | 1606,84 | 5,26 | 0,79 | Silica | Sigma-Alrdich | 49,00 | 2,00 |
| Exp. 6 | 397,0 | 110 | 5 | 6 | 85,10 | 100,00 | 2,18 | 1633,71 | 5,26 | 0,79 | Silica | Sigma-Alrdich | 49,00 | 2,00 |
| Exp. 6 | 404,5 | 110 | 5 | 6 | 84,20 | 100,00 | 2,18 | 1616,44 | 5,26 | 0,79 | Silica | Sigma-Alrdich | 49,00 | 2,00 |
| Exp. 6 | 423,0 | 110 | 1 | 2 | 96,80 | 100,00 | 8,55 | 483,16 | 5,26 | 0,79 | Silica | Sigma-Alrdich | 49,00 | 2,00 |
| Exp. 6 | 496,5 | 110 | 1 | 2 | 97,90 | 100,00 | 8,55 | 488,66 | 5,26 | 0,79 | Silica | Sigma-Alrdich | 49,00 | 2,00 |
| Exp. 7 | 3,0 | 150 | 5 | 6 | 4,80 | 100,00 | 1,16 | 172,80 | 2,84 | 0,80 | Alfa - Al2O3 | SASOL | 17.7 - 20.9 | 0.67 |
| Exp. 8 | 5,0 | 150 | 5 | 6 | 5,80 | 100,00 | 0,70 | 346,33 | 3,06 | 1,43 | Glass Beads | Sigma-Alrdich | 1.95 - 4.07 | 0.07 |
| Exp. 9 | 3,5 | 150 | 5 | 6 | 22,20 | 100,00 | 0,74 | 1260,31 | 2,70 | 1,20 | SiC | Sigma-Alrdich | 13.5 - 16.3 | 0.64 |
| Exp. 9 | 22,0 | 150 | 5 | 6 | 21,60 | 100,00 | 0,74 | 1226,25 | 2,70 | 1,20 | SiC | Sigma-Alrdich | 13.5 - 16.3 | 0.64 |
| Exp. 9 | 46,0 | 150 | 5 | 6 | 20,60 | 100,00 | 0,74 | 1169,48 | 2,70 | 1,20 | SiC | Sigma-Alrdich | 13.5 - 16.3 | 0.64 |
| Exp. 10 | 1,5 | 150 | 5 | 6 | 32,70 | 100,00 | 0,95 | 1434,24 | 3,32 | 1,14 | Al2O3 | Sigma-Alrdich | 16.4 - 20.2 | 0.77 |
| Exp. 10 | 20,0 | 150 | 5 | 6 | 30,00 | 100,00 | 0,95 | 1315,82 | 3,32 | 1,14 | Al2O3 | Sigma-Alrdich | 16.4 - 20.2 | 0.77 |

Table 2

| Exp. N° | BET (m2/g) | Pore volume (macro) (cm3/g) | Pore volume (meso) (cm3/g) |
|---|---|---|---|
| 7 | 3.7 | 0.18 | Below Limit of detection |
| 8 | <0.1 | 0.02 | Below Limit of detection |
| 9 | 0.3 | 0.37 | Below Limit of detection |
| 1 to 6 | 347 | 2.06 | 0.99 |
| 10 | 3 | 0.55 | <0.01 |

# Table 3

| Exp | Points | T, °C | C2H2, LN/h | HCl, LN/h | p, bar | Density, kg/m3 | Mass flow, 10-6 kg/s | length, m | delta p, kPa | sp, delta p KPa/m | v, m/s |
|---|---|---|---|---|---|---|---|---|---|---|---|
| 1 | a,b | 110 | 5 | 6 | 1 | 1,01 | 4,32 | 0,078 | 1,6 | 20,7 | 0,05 |
| | c,d | 150 | 5 | 6 | 1 | 0,91 | 4,32 | 0,078 | 1,8 | 23 | 0,06 |
| 2 | a,b,c | 150 | 5 | 6 | 1 | 0,91 | 4,32 | 0,082 | 1,9 | 23 | 0,06 |
| 3 | a,b,c,d | 110 | 5 | 6 | 1 | 1,01 | 4,32 | 0,086 | 1,8 | 20,7 | 0,05 |
| 4 | a,b | 90 | 5 | 6 | 1 | 1,06 | 4,32 | 0,179 | 3,5 | 19,8 | 0,05 |
| | reste | 110 | 5 | 6 | 1 | 1,01 | 4,32 | 0,179 | 3,7 | 20,7 | 0,05 |
| 5 | a,f | 110 | 5 | 6 | 1 | 1,01 | 4,32 | 0,226 | 4,7 | 20,7 | 0,05 |
| | g,i | 110 | 1 | 2 | 1 | 1,05 | 1,23 | 0,226 | 1,2 | 5,3 | 0,01 |
| 6 | a,q | 110 | 5 | 6 | 1 | 1,01 | 4,32 | 0,119 | 2,5 | 20,7 | 0,05 |
| | r,s | 110 | 1 | 2 | 1 | 1,05 | 1,23 | 0,119 | 0,6 | 5,3 | 0,01 |

Table 4

| Exp. N | SILP | T | t | Corrosion | Catalyst used | |
|---|---|---|---|---|---|---|
| | | /°C | /h | /µm/year | Description | Reference to experiment table 1 |
| 11 | NO: Blank reaction Zr + HCl + $C_2H_2$, No SILP no | 150 | 2 | 0,00 | No catalyst used | n/a |
| 12 | NO: Controlling experiment: Pd/$SiO_2$ (no IL) | 150 | 2 | 0,00 | 2 w/w % Pd on $SiO_2$ (Sigma Aldrich) withouth | same catalyst used for exp. 1 |
| 13 | YES: 50% Pore volume filled | 110 | 2 | 0,00 | SILP: 1,3 w/w % Pd, 33 w/w % IL on $SiO_2$ | n/a |
| 14 | YES: 50% Pore volume filled | 130 | 2 | 460,87 | SILP: 1,3 w/w % Pd, 33 w/w % IL on $SiO_2$ | n/a |
| 15 | YES: 50% Pore volume filled | 150 | 2 | 3301,22 | SILP: 1,3 w/w % Pd, 33 w/w % IL on $SiO_2$ | n/a |
| 16 | YES: 90% Pore volume filled | 110 | 2 | 18468,88 | SILP: 1 w/w % Pd, 49 w/w % IL on $SiO_2$ | same catalyst used for Exp 4, Exp |
| 17 | NO: IONIC LIQUID: BminCl + $PdCl_2$ | 110 | 2 | 850356,62 | IL system: 15 mL Bmim Cl + $PdCl_2$ (22,6 mM) | n/a |
| 18 | NO: IONIC LIQUID: BminCl + $PdCl_2$ | 130 | 2 | (completely dissolved) | IL system: 15 mL Bmim Cl + $PdCl_2$ (22,6 mM) | n/a |

Table 5

| S/V | D of particles, m | C2H2,/HCl mol/m | T, °C | p, MPa | density,kg/m3 | v, m/s | mass flow, kg/s | sp deltap, kPa/m |
|---|---|---|---|---|---|---|---|---|
| 1000 | 0,006 | 0,8 | 100 | 0,1 | 1,04 | 1 | 0,815 | 3,1 |
| | | 0,8 | 100 | 0,2 | 2,08 | 1 | 1,63 | 5,9 |
| | | 0,00015 | -30 | 1,2 | 21,92 | 0,5 | 8,6 | 15,1 |
| 10000 | 0,0006 | 0,8 | 100 | 0,1 | 1,04 | 0,7 | 0,57 | 30,2 |
| | | 0,8 | 100 | 0,2 | 2,08 | 0,5 | 0,815 | 25,8 |
| | | 0,00015 | -30 | 1,2 | 21,92 | 0,2 | 3,44 | 28,6 |
| 100000 | 0,00006 | 0,8 | 100 | 0,2 | 2,08 | 0,05 | 0,08 | 116,1 |
| | | 0,00015 | -30 | 1,2 | 21,92 | 0,03 | 0,5 | 73,2 |

Europäisches Patentamt
European Patent Office
Office européen des brevets

# EUROPEAN SEARCH REPORT

Application Number
EP 12 17 3956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 2008/077868 A1 (SOLVAY [BE]; PETITJEAN ANDRE [BE]; STREBELLE MICHEL [BE]; DEVOS ANDRE) 3 July 2008 (2008-07-03)<br>* the whole document *<br>----- | 1-12,14, 15<br>13 | INV.<br>C07C17/08<br>C07C21/06 |
| Y,D<br><br>A | DATABASE WPI<br>Week 201043<br>Thomson Scientific, London, GB;<br>AN 2010-G98169<br>XP002687039,<br>& CN 101 716 528 A (YU Z)<br>2 June 2010 (2010-06-02)<br>* abstract *<br>----- | 13<br><br>1-12,14, 15 | |

TECHNICAL FIELDS SEARCHED (IPC)
C07C

The present search report has been drawn up for all claims

3

| Place of search | Date of completion of the search | Examiner |
|---|---|---|
| The Hague | 18 December 2012 | Mooren, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 17 3956

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2012

| Patent document cited in search report | Publication date | Patent family member(s) | Publication date |
|---|---|---|---|
| WO 2008077868 A1 | 03-07-2008 | AR 064514 A1 | 08-04-2009 |
| | | AU 2007338081 A1 | 03-07-2008 |
| | | CN 101605604 A | 16-12-2009 |
| | | CO 6210696 A2 | 20-10-2010 |
| | | EP 2101916 A1 | 23-09-2009 |
| | | FR 2910350 A1 | 27-06-2008 |
| | | RU 2009128222 A | 27-01-2011 |
| | | TW 200835553 A | 01-09-2008 |
| | | US 2010063333 A1 | 11-03-2010 |
| | | WO 2008077868 A1 | 03-07-2008 |
| | | ZA 200904659 A | 29-09-2010 |
| CN 101716528 A | 02-06-2010 | CN 101716528 A | 02-06-2010 |
| | | WO 2011050614 A1 | 05-05-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 52136104 A
- DE 709000
- SU 237116
- EP 0340416 A
- EP 0519548 A1
- EP 0525843 A1
- WO 200877868 A
- EP 0519548 A
- EP 0525843 A
- CN 101716528
- EP 2012052911 W

### Non-patent literature cited in the description

- **ZHIYONG YU.** *Green Chem.,* 2011, vol. 13, 1495